Europäisches Patentamt

European Patent Office

Office européen des brevets

(11) Veröffentlichungsnummer : **0 100 109**
**B1**

# EUROPÄISCHE PATENTSCHRIFT

(45) Veröffentlichungstag der Patentschrift :
**11.12.85**

(21) Anmeldenummer : **83200531.8**

(22) Anmeldetag : **14.04.83**

(51) Int. Cl.⁴ : **C 07 D215/04// B01D3/36**

(54) **Verfahren zur Isolierung von Basen aus Gemischen mit aromatischen Kohlenwasserstoffen.**

(30) Priorität : **24.07.82 DE 3227759**

(43) Veröffentlichungstag der Anmeldung :
**08.02.84 Patentblatt 84/06**

(45) Bekanntmachung des Hinweises auf die Patenterteilung : **11.12.85 Patentblatt 85/50**

(84) Benannte Vertragsstaaten :
**DE FR GB IT**

(56) Entgegenhaltungen :
**DE-A- 2 123 347**
**DE-B- 812 079**
**DE-B- 832 155**
**GB-A- 2 007 650**
**US-A- 2 999 794**
**CHEMICAL ABSTRACTS, Band 76, 1972, Spalte 36022, Nr. 36020q, Columbus, Ohio, USA G.B. LEK-HOVA et al.: "Azeotropy in binary and ternary systems formed by some components of coal tar"**
**CHEMICAL ABSTRACTS, Band 76, 1972, Spalte 36022, Nr. 36021r, Columbus, Ohio, USA G.B. LEK-HOVA et al.: "Azeotropy in binary systems formed by some components of coal tar"**
**CHEMICAL ABSTRACTS, Band 76, 1972, Spalte 36022, Nr. 36022s, Columbus, Ohio, USA G.B. LEK-HOVA et al.: "Azeotropy in ternary systems formed by some components of coal tar"**
**CHEMISTRY OF HETEROCYCLIC COMPOUNDS, Band 32, Teil 1, 1977, Seiten 2-68, "The physical and chemical properties of quinoline", John Wiley & Sons, New York, USA**

(73) Patentinhaber : **Rütgerswerke Aktiengesellschaft Mainzer Landstrasse 217 D-6000 Frankfurt a.Main 1 (DE)**

(72) Erfinder : **Hörmeyer, Heinrich, Dr. Propsteistrasse 14 D-4300 Essen 16 (DE)**

## Beschreibung

Die Erfindung betrifft ein Verfahren zur Abtrennung von Chinolinbasen aus ihren Gemischen mit aromatischen Kohlenwasserstoffen.

Bisher werden die Basen aus Teerölen in technischem Maßstab fast ausschließlich durch Extraktion mit wässrigen Säuren, zumeist Schwefelsäure, anschließendes Klardampfen, Neutralisation mit anorganischen Basen, Entwässerung und fraktionierte Destillation der abgetrennten Teerbasen gewonnen.

Ein Verfahren zur Gewinnung stickstoffhaltiger aromatischer Kohlenwasserstoffe durch selektive Elution von einem anorganischen Träger ist Gegenstand einer älteren Anmeldung (DE-31 14 346 A1) der Anmelderin. Das Verfahren ist aufwendig, da große Lösungsmittelmengen aufgearbeitet werden müssen.

An alle Verfahren muß ein strenger Maßstab bezüglich der Reinheit, insbesondere der Neutralölfreiheit der Basen gelegt werden.

Zur Gewinnung von aromatischen Stickstoffverbindungen, die einen Pyrrolring enthalten, wurden auch azeotrope Trennverfahren beschrieben. Beispielsweise läßt sich ein Zweistoffgemisch von Biphenyl und Indol (DE-PS 812 079 und DE-PS 832 155) oder ein Dreistoffgemisch aus Anthracen, Phenanthren und Carbazol (DE-OS 2 123 342) durch azeotrope Destillation trennen. Ethylenglykol wird zwar in beiden Fällen auch als mögliches Schleppmittel angeführt. Im ersten Fall handelt es sich um Diethylenglykol ; im zweiten Fall ist aus den Beispielen zu entnehmen, daß die Wirksamkeit gegenüber den anderen Schleppmitteln wie Ethylencarbonat und Propylencarbonat wesentlich geringer ist.

Ein Verfahren für eine nahezu quantitative Schleppmittel-Rückgewinnung wurde nicht entwickelt und eine Anwendung der Verfahren auf Fraktionen mit geringeren Gehalten an Stickstoffverbindungen für nicht günstig gehalten. Insbesondere wurde eine Abtrennbarkeit der in chemischer Hinsicht vollkommen verschiedenen Basen mit Pyridinringsystemen von ihren Siedebegleitern nicht berücksichtigt.

Weitere Verfahren wurden für die Auftrennung der Basenfraktionen entwickelt, die bereits durch aufwendige Methoden von neutralen und sauren Kohlenwasserstoffen befreit wurden.

So kann beispielsweise die Trennung zwischen Chinolin und Isochinolin auch durch azeotrope Destillation mit Diethylenglykol erfolgen (N.D. Russjanova, M.W. Goftman, Wiss. Hochschulber., Chem. Technol. (UdSSR) 1959, 376-79).

Es ist auch bekannt, das Isochinolin als saures Sulfit von Chinolin abzutrennen (M. M. Potaschnikow, J. angew. Chem. 32, 428-32, Febr. 1959).

Beide Verfahren besitzen keine technische Bedeutung, da die Trennung zwischen Chinolin und Isochinolin billiger schon durch einfache Vakuumrektifikation gelingt. Im übrigen ist bei beiden Verfahren die Entbasung durch Mineralsäuren nach wie vor erforderlich.

Auch für die Reingewinnung anderer Basen können oder müssen andere als rein destillative Verfahren eingesetzt werden (H.-G. Franck, G. Collin, Steinkohlenteer, Berlin-Heidelberg 1968, S. 62-64 und S. 84-88). In jedem Fall geht der Auftrennung des Basengemisches eine anorganisch-chemische Entbasung der Aromatenfraktion voraus.

Alle vorstehenden und bekannten Verfahrensvarianten haben den Nachteil, daß für das komplizierte Aufarbeitungsschema ein hoher apparativer und personeller Aufwand benötigt wird und daß große Mengen Abwasser und andere Nebenprodukte entsorgt werden müssen.

Es bestand daher die Aufgabe, ein Verfahren zur nahezu quantitativen Abtrennung von Chinolinbasen aus einem Gemisch mit aromatischen Kohlenwasserstoffen zu entwickeln, das die Nachteile der aufgeführten bekannten Verfahren vermeidet und auch auf Gemische mit geringem Basengehalt anwendbar ist.

Die Aufgabe wurde dadurch gelöst, daß das basenhaltige Aromatengemisch einer azeotropen Destillation mit Monoethylenglykol als Schleppmitel unterworfen wird, wobei das kondensierte Destillat durch Dekantieren in eine Aromaten- und eine Glykolfraktion aufgetrennt, die Aromatenfraktion ausgeschleust und die Glykolfraktion in die Kolonne zurückgeführt wird, anschließend der aus einem Basen/Glykol-Gemisch bestehende Destillationsrückstand zur Gewinnung der reinen Basenfraktion destillativ aufgearbeitet und gegebenenfalls die Basenfraktion in die einzelnen Basen aufgetrennt wird.

Als basenhaltige Aromatengemische sind vor allem Fraktionen aus der Aufarbeitung von Kohleveredlungsprodukten, insbesondere Steinkohlenteeröle, wie z. B. Methylnaphthalinöl und die Biphenyl-Indol-Fraktion (Franck/Collin, Steinkohlenteer, Springer-Verlag 1968, S. 60) anzusehen.

Nach einer vorteilhaften Ausführung des Verfahrens wird 1 Teil Einsatzgemisch unter Zusatz von 1 bis 2 Teilen Monoethylenglykol destillativ so aufgearbeitet, daß am Kopf der Kolonne eine Kohlenwasserstoff-/Glykolfraktion abgenommen wird. Nach ihrer Kondensation bilden sich zwei Phasen. Die Glykolphase wird in die Kolonne zurückgegeben, die Kohlenwasserstoffe werden ausgeschleust. Nach Abtrennung der Kohlenwasserstoffe bleiben Basen und Glykol im Sumpfprodukt zurück.

Diese erste Destillationsstufe wird vorzugsweise in Kolonnen mit 5 bis 30 theoretischen Böden durchgeführt. Das Rücklaufverhältnis Rücklauf zu Destillat kann R/D = 0,5 : 1 bis 5 : 1 betragen. Der Basengehalt in der Fraktion der aromatischen Kohlenwasserstoffe beträgt zwischen 0,2 und 2 Gew.-%. Der Basengehalt der zirkulierenden Glykolphase liegt zwischen 0 und 5 %. Das Verhältnis von Glykol zu aromatischen Kohlenwasserstoffen im Destillat kann je nach der mittleren Siedepunktdifferenz zwischen Schleppmittel und Einsatzmaterial von 1 : 1 bis 2,5 : 1 liegen.

In einem zweiten Schritt wird danach das Glykol von den im Sumpfprodukt zurückgebliebenen Basen destillativ abgetrennt. Dabei können zusammen mit dem Glykol noch Spuren von Aromaten im Kopfprodukt erhalten werden, die der Reingewinnung der Basen hinderlich gewesen wären.

Diese Destillation wird vorzugsweise in Kolonnen mit 10 bis 60 theoretischen Böden und mit einem Rücklaufverhältnis R/D = 2 : 1 bis 10 : 1 durchgeführt. Der Basengehalt in der abgetrennten Glykolphase kann bis zu 33 % betragen. Es ist daher sinnvoll, diese Glykolphase statt des reinen Monoäthylenglykols dem Einsatzgemisch zuzusetzen.

Im dritten Schritt werden aus dem Sumpfrückstand die Basen rein gewonnen. Dies kann nach jedem der bekannten Verfahren zur Auftrennung von Basengemischen geschehen, im Falle der Chinolinbasen vorzugsweise destillativ. Das Chinolin kann direkt destillativ von den anderen Basen abgetrennt werden.

Die Reingewinnung von Chinaldin, Isochinolin und Lepidin wird durch Redestillation von Fraktionen erreicht, in denen sie angereichert vorliegen. Kolonnenhöhen und Rücklaufverhältnisse können wie bei der bekannten Basenaufarbeitung gewählt werden.

In einer besonders vorteilhaften Ausführungsform (Fig.) werden zumindest die beiden ersten Aufarbeitungsschritte hintereinander in zwei kontinuierlich betriebenen Destillationskolonnen durchgeführt, wobei die Betriebsdrücke und Rücklaufverhältnisse der Kolonnen jeweils so eingestellt werden, daß mit den Brüden der ersten Kolonne die erforderliche Energie für den Betrieb der zweiten Kolonne auf ausreichendem Temperaturniveau bereitgestellt werden kann. Dabei wird das Einsatzmaterial über die Leitung 5 unter Zusatz rückgeführten Glykols in die Mitte der kontinuierlichen Kohlenwasserstoffkolonne 1 eingespeist, am Kopf derselben das Kondensat im Phasenscheider 4 in eine Kohlenwasserstoff- und in eine Glykolphase getrennt, die Kohlenwasserstoffe werden über die Leitung 7 ausgeschleust und das Glykol wird über die Leitung 8 dem Einsatz wieder zugeführt. Am Sumpf der Kolonne 1 wird ein Basen-/Glykol-Gemisch über den Stutzen 10 abgenommen und eventuell unter dem Zusatz geringer Glykolmengen, die bei der Reingewinnung der Basen anfallen, in die Mitte der kontinuierlichen Glykolkolonne 2 eingespeist, wo die zweite Aufarbeitungsstufe durchgeführt wird. Das basenreiche Destillat wird über die Leitung 9 wieder zur Einspeisestelle der ersten Kolonne zurückgeführt und mit den beiden anderen Produktströmen vermischt. Der Rückstand wird bei einem Restgehalt von 0-1,5 % Glykol in der Kolonne 3 auf das Reinprodukt bzw. die Reinprodukte aufgearbeitet. Dazu wird er über die Leitung 12 in die Mitte der Basenkolonne 3 kontinuierlich eingespeist. Die geringen Glykolmengen werden, falls vorhanden, über die Leitung 11 dem Einsatz für die Kolonne 2 zugemischt.

Die Chinolinfraktion wird an der Entnahmestelle 13 und die Chinaldinfraktion an der Entnahmestelle 14 abgezogen. Der Rückstand wird über den Stutzen 15 abgenommen. Es ist ebenso gut möglich, die Trennung der Basenfraktion (Rückstand der Kolonne 2) zu speichern und diskontinuierlich zu fraktionieren.

Die Azeotropie von Glykol mit den Hauptkomponenten des Methylnaphthalinöles ist bekannt (L.H. Horsley, Azeotropic Data, Washington, D.C., 1952 ; H.-G. Franck, Brennstoff-Chemie, 32, 199 (1951). Dabei wurde aber nicht erkannt, daß Chinolin gegenüber den Kohlenwasserstoffen in Gegenwart von Glykol ein sehr verschiedenes Siedeverhalten zeigt. Auch wurden stets nur binäre oder ternäre Azeotrope untersucht. Die Möglichkeit der Reingewinnung der Basen durch azeotrope Destillation eines Basen-, Kohlenwasserstoffgemisches oder gar direkt aus einer Teerölfraktion wurde nicht untersucht.

Das erfindungsgemäße Verfahren zeichnet sich gegenüber bestehenden Basengewinnungsverfahren durch eine Reihe von Vorteilen aus :

1. Die Ausbeute an Teerbasen wird gesteigert.

2. Die Produktgewinnung erfolgt rein destillativ und ohne Einsatz anorganischer Stoffe.

3. Dadurch entstehen keine Kosten durch Abwässer und Nebenprodukt-Aufarbeitung.

4. Das Verfahren läßt sich vollkontinuierlich und bei weitgehender Wärmerückgewinnung realisieren.

Das vorgeschlagene Verfahren läßt sich zur Gewinnung einer großen Anzahl von Stoffen anwenden. Als Beispiele können ohne Anspruch auf Vollständigkeit genannt werden : Chinolin, Isochinolin, Chinaldin und Lepidin.

Die Erfindung wird anhand der Beispiele 1 bis 3 näher erläutert. Das Beispiel 4 ist ein Vergleichsbeispiel zu Beispiel 3, bei dem statt Monoethylenglykol ein anderes Schleppmittel, Propylencarbonat, wie in der DE-OS 21 23 342 vorgeschlagen, verwendet wurde. Das Vergleichsbeispiel zeigt, daß die für die Gewinnung stickstoffhaltiger Aromaten, die einen Pymolring enthalten, vorgeschlagenen besonders vorteilhaften Schleppmittel nicht ebenso für die Abtrennung von Chinolinbasen geeignet sind.

Beispiel 1

Eine Methylnaphthalinöl aus Steinkohlenteer mit einem Gehalt an Chinolin von 7,5 %, an Isochinolin von 1,8 %, an Chinaldin von 1,0 % wird unter Zusatz von 10 % Monoethylenglykol in einer zehnbödigen Glockenboden-Kolonne bei einem Rücklaufverhältnis von R/D = 5 : 1 rektifiziert.

Das Glykol wird dabei mit Hilfe eines Phasenscheiders nach Kondensation am Kopf der Kolonne von der Ölphase abgetrennt und direkt in den Sumpf zurückgegeben. Nachdem 82 % des eingesetzten Öles überdestilliert sind, zeigt sich im Kopfprodukt eine Anreicherung von Chinolin. Die Destillation wird daraufhin abgebrochen und das gesamte Glykol mit dem Sumpfprodukt vereinigt.

Das Sumpfprodukt der ersten Kolonne wird nun in einer Füllkörpersäule mit ca. 40 theoretischen Böden weiter bei einem Rücklaufverhältnis von R/D = 30 : 1 destilliert. Bei 70 mbar Kopfdruck wird zunächst den Siedepunkten entsprechend eine Glykolfraktion abgenommen. Nach einem Sprung in der Kopftemperatur von 125,5 °C auf 146 °C wird ein Chinolin in einer Reinheit von über 99 % gewonnen. Danach erfolgt eine Isochinolin-Chinaldinfraktion.

Die Chinolinausbeute der ersten Destillation beträgt 93,2 %, die der zweiten 88,2 %, so daß sich für Chinolin eine Gesamtausbeute von 82,2 % ergibt.

Die Isochinolin-Chinaldinausbeute beträgt 53,6 % bei einer Anreicherung beider Komponenten auf 80,1 %.

Beispiel 2

Ein Methylnaphthalinöl aus der Steinkohlenteerdestillation wird zur Gewinnung der Basen in zwei kontinuierlichen und einer diskontinuierlichen Kolonne aufgearbeitet.

350 Gewichtsteile Methylnaphthalinöl mit einem Chinolingehalt von 7,9 % einem Isochinolingehalt von 1,9 % und einem Chinaldingehalt von 1,2 % unter Zusatz von 650 Gewichtsteilen glykolhaltigen Destillats aus dem Phasenscheider der Kohlenwasserstoffkolonne und der Glykolkolonne, das zu 94 % aus Monoäthylenglykol besteht, werden in die Kohlenwasserstoffkolonne eingespeist, die aus einem Verstärker- und einem Abtriebsteil von je 15 Glockenböden besteht und bei 1 bar Kopfdruck betrieben wird.

Am Kopf der Kolonne wird das Kondensat im Phasenscheider in eine Öl- und eine Glykolphase getrennt. Die Glykolphase wird rezirkuliert, die Ölphase mit einem Restgehalt von 0,3 % Chinolin aus dem Phasenscheider entnommen.

213 Gewichtsteile Sumpf werden unter Zusatz von 3 Gewichtsteilen Glykolphase aus der Basenkolonne in die Glykolkolonne gespeist. Dort werden die restlichen Kohlenwasserstoffe aus dem Siedebereich des Methylnaphthalinöles bei 100 mbar Kopfdruck von den Basen vollständig abgetrennt.

Die Glykolkolonne besitzt 15 Verstärker- und 15 Abtriebsböden. Das Kopfprodukt wird in die Kohlenwasserstoff-Kolonne zurückgeführt, der Sumpfrückstand wird gesammelt und zur diskontinuierlichen Basendestillation in die Basenkolonne 3 gegeben. Dort werden in einer Füllkörpersäule mit ca. 40 theoretischen Böden aus 60 Gewichtsteilen Einsatzmaterial bei einem Rücklaufverhältnis von R/D = 30 : 1 3 Gewichtsteile Glykolvorlauf, 22,7 Gewichtsteile Chinolin in einer Reinheit von 97,4 %, 10,3 Gewichtsteile Chinolin-Chinaldinfraktion in einer Reinheit von 70,8 % und 24 Gewichtsteile Sumpfrückstand abgenommen.

Die Gesamtausbeute an Chinolin beträgt mithin 80,0 %, die an Chinolin und Chinaldin in ihrer gemeinsamen Fraktion 67,2 %.

Bei den gewählten Betriebsdrücken ist die Brüdentemperatur am Kopf der Kohlenwasserstoff-Kolonne so hoch, daß es möglich ist, mit den Brüden der Kohlenwasserstoff-Kolonne den gesamten Wärmebedarf der Glykol-Kolonne zu decken und auch noch das Einsatzmaterial vorzuwärmen.

Beispiel 3

Ein Gemisch aus 1 Gewichtsteil Chinaldin (Siedepunkt 248 °C), 1 Gewichtsteil Biphenyl (Siedepunkt 255 °C) und 3 Gewichtsteilen Monoethylenglykol wird in einer zehnbödigen Siebbodenkolonne diskontinuierlich bei einem Rücklaufverhältnis von R/D = 2 : 1 und 1 bar Kopfdruck destilliert.

Am Kopf werden zunächst 3,10 Gewichtsteile eines Destillates abgenommen, das sich oberhalb des Schmelzpunktes von Biphenyl (69,2 °C) in 0,87 Gewichtsteile Öl- und 2,23 Gewichtsteile Glykolphase trennen läßt. Die Ölphase besteht zu 94,9 % aus Biphenyl.

Nach einer Zwischenfraktion von 1,13 Gewichtsteilen, die sich in 0,08 Gewichtsteile Öl- und 1,05 Gewichtsteile Glykolphase trennen läßt und deren Ölphase zu 57,2 % aus Chinaldin und zu 42,8 % aus Biphenyl besteht, lassen sich noch 0,52 Gewichtsteile eines zu 97,5 % reinen Chinaldins destillieren. 0,18 Gewichtsteile eines zu 94,6 % reinen Chinaldins werden in der Kolonne zurückgehalten und als Sumpfrückstand entnommen.

Schon ohne Berücksichtigung einer Zirkulation der Zwischenfraktion beträgt damit die Ausbeute an Chinaldin 50,7 % und an Diphenyl 82,6 %.

Bei Zirkulation des gesamten Glykols und der Ölphase der Zwischenfraktion erhöht sich die Ausbeute an Chinaldin und Biphenyl auf ungefähr 95 %.

Beispiel 4 (Vergleichsbeispiel)

Ein Gemisch aus gleichfalls 1 Gewichtsteil Chinaldin und 1 Gewichtsteil Biphenyl wird wie in Beispiel 3, jedoch mit 3 Gewichtsteilen Propylencarbonat (Siedepunkt 243 °C), in einer zehnböden Siebbodenkolonne diskontinuierlich bei einem Rücklaufverhältnis von R/D = 2 : 1 und 1 bar Kopfdruck destilliert.

Am Kopf werden zunächst 1,83 Gewichtsteile einer auch bei Raumtemperatur noch homogenen Fraktion aus 27 % Chinaldin, 19 % Biphenyl und 54 % Propylencarbonat abgenommen.

Darauf folgen als ebenfalls homogene Phase 1,80 Gewichtsteile aus 22 % Chinaldin, 23 % Biphenyl und 55 % Propylencarbonat.

Zurück bleiben schließlich 1,2 Gewichtsteile mit 9,5 % Chinaldin, 16,5 % Biphenyl und 74 % Propylencarbonat.

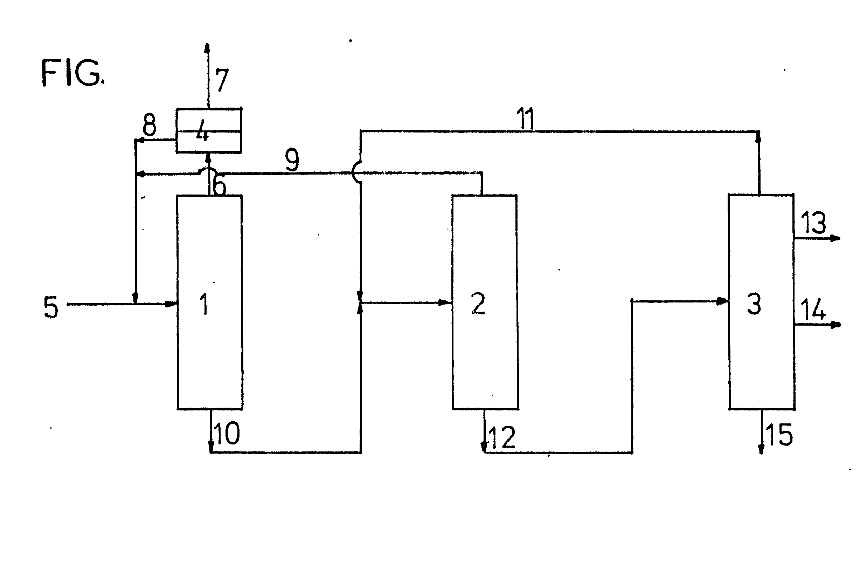

**Patentansprüche**

1. Verfahren zur Abtrennung von Chinolinbasen aus ihren Gemischen mit aromatischen Kohlenwasserstoffen, dadurch gekennzeichnet, daß das Aromatengemisch einer azeotropen Destillation mit Monoäthylenglykol als Schleppmittel unterworfen wird, wobei das kondensierte Destillat durch Dekantieren in eine Aromaten- und eine Glykolfraktion aufgetrennt, die Aromatenfraktion ausgeschleust und die Glykolfraktion in die Kolonne zurückgeführt wird, anschließend der aus einem Basen/Glykol-Gemisch bestehende Destillationsrückstand zur Gewinnung der reinen Basenfraktion destillativ aufgearbeitet und gegebenenfalls die Basenfraktion in die Basen aufgetrennt wird.

2. Verfahren nach Anspruch 1, dadurch gekennzeichnet, daß die azeotrope Destillation in einer Kolonne mit 5 bis 30 theoretischen Böden unter einem Rücklaufverhältnis R/D von 1 : 2 bis 5 : 1 durchgeführt wird.

3. Verfahren nach Anspruch 1, dadurch gekennzeichnet, daß die Destillation zur Trennung des Basen/Glykol-Gemisches in einer Kolonne mit 10 bis 60 theoretischen Böden unter einem Rücklaufverhältnis R/D von 2 : 1 bis 10 : 1 durchgeführt wird.

4. Verfahren nach Anspruch 1, dadurch gekennzeichnet, daß zumindest die ersten beiden Aufarbeitungsschritte hintereinander in zwei kontinuierlich betriebenen Destillationskolonnen durchgeführt werden, wobei die Betriebsdrücke und Rücklaufverhältnisse so eingestellt werden, daß mit den Brüden der ersten Kolonne die erforderliche Energie für den Betrieb der zweiten Kolonne bereitgestellt wird.

### Claims

1. Process for separating of quinoline bases from their mixture with aromatic hydrocarbons characterised by an azeotropic distillation of the aromatic mixture with monoethylene glycol as carrier, wherein the condensed distillate is separated into an aromatic fraction and a glycol fraction by decantation, the aromatic fraction is removed and the glycol fraction is recycled into the distillation tower, distilling the residue from the foregoing azeotropic distillation for the recovering of a pure fraction of the quinoline bases from this mixture of the glycol and the bases and optionally separating this fraction in the different bases.

2. Process as claimed in claim 1, wherein the azeotropic distillation is carried out in a tower with 5 to 30 theoretical plates with a reflux ratio R/D within the range from 1 : 2 to 5 : 1.

3. Process as claimed in claim 1, wherein the distillation of the residue for separating the mixture of bases and glycol is carried out in a tower with 10 to 60 theoretical plates with a reflux ratio within the range from 2 : 1 to 10 : 1.

4. Process as claimed in claim 1, wherein at least the first two steps are carried out one after the other in two continuously working distillation towers under such a pressure and reflux ratio that the vapors of the first tower provide the second tower with the necessary energy.

### Revendications

1. Procédé pour la séparation de bases quinoléiniques et de leurs mélanges avec des hydrocarbures aromatiques, caractérisé en ce que le mélange de composés aromatiques est soumis à une distillation azéotropique avec le monoéthylèneglycol en tant qu'agent d'entraînement, à l'occasion de quoi le distillat condensé est séparé par décantation en une fraction de composés aromatiques·et une fraction de glycol, la fraction de composés aromatiques étant prélevée et la fraction de glycol ramenée dans la colonne, le résidu de_ distillation constitué d'un mélange bases/glycol étant ensuite traité par distillation pour obtenir la fraction de bases pure et la fraction de bases étant éventuellement séparée de manière en soi connue en les bases.

2. Procédé selon la revendication 1, caractérisé en ce que la distillation azéotropique est effectuée dans une colonne avec 5 à 30 plateaux théoriques sous un taux de retour R/D de 1 : 2 à 5 : 1.

3. Procédé selon la revendication 1, caractérisé en ce que la distillation pour séparer le mélange bases/glycol est effectuée dans une colonne ayant 10 à 60 plateaux théoriques sous un taux de retour R/D de 2 : 1 à 10 : 1.

4. Procédé selon la revendication 1, caractérisé en ce qu'au moins les deux premières étapes de traitement sont effectuées successivement dans deux colonnes de distillation fonctionnant en continu, les pressions de fonctionnement et les taux de retour étant réglés de sorte que l'énergie nécessaire au fonctionnement de la deuxième colonne soit mise à disposition au moyen des vapeurs chaudes de la première colonne.

FIG.

0 100 109